Europäisches Patentamt

European Patent Office

Office européen des brevets

(19)

(11) Publication number: 0 317 085
A2

(12) **EUROPEAN PATENT APPLICATION**

(21) Application number: 88309849.3

(22) Date of filing: 20.10.88

(51) Int. Cl.⁴: **G01N 33/556 , G01N 33/96 , G01N 33/80**

(30) Priority: **20.10.87 JP 265914/87**

(43) Date of publication of application:
**24.05.89 Bulletin 89/21**

(84) Designated Contracting States:
**DE FR GB IT**

(71) Applicant: **SEITETSU KAGAKU CO., LTD.**
**346-1, Miyanishi Harimacho, Kako-gun**
**Hyogo-ken 673-01(JP)**

(72) Inventor: **Fukuda, Hirosuke c/o Seitetsu**
**Kagaku Co., Ltd.**
**Research Laboratory 346-1, Miyanishi**
**Harima-cho**
**Kako-gun Hyogo-ken(JP)**
Inventor: **Kawamura, Masao c/o Seitetsu**
**Kagaku Co., Ltd.**
**Research Laboratory 346-1, Miyanishi**
**Harima-cho**
**Kako-gun Hyogo-ken(JP)**
Inventor: **Akutsu, Seiichi c/o Seitetsu Kagaku**
**Co., Ltd.**
**Research Laboratory 346-1, Miyanishi**
**Harima-cho**
**Kako-gun Hyogo-ken(JP)**
Inventor: **Saga, Kouichi c/o Seitetsu Kagaku**
**Co., Ltd.**
**Research Laboratory 346-1, Miyanishi**
**Harima-cho**
**Kako-gun Hyogo-ken(JP)**
Inventor: **Morimoto, Shigeru c/o Seitetsu**
**Kagaku Co., Ltd.**
**Research Laboratory 346-1, Miyanishi**
**Harima-cho**
**Kako-gun Hyogo-ken(JP)**
Inventor: **Matsuzawa, Shigetaka**
**1-5-16, Motoizumi**
**Komae-shi Tokyo-to(JP)**
Inventor: **Seno, Taiko**
**3-5-16, Nakahama Joto-ku**
**Osaka-shi Osaka-fu(JP)**
Inventor: **Okubo, Yasuto**
**3-11-18, Taishi Ohji-cho**
**Kitakatsuragi-gun Nara-ken(JP)**

(74) Representative: **Armitage, Ian Michael et al**
**MEWBURN ELLIS & CO. 2/3 Cursitor Street**
**London EC4A 1BQ(GB)**

EP 0 317 085 A2

(54) **Processed red blood cells and process for producing the same.**

(57) Processed red blood cells having storage stability with maintaining original antigenicity of natural natural red blood cells which is obtained by mild treatment of natural red blood cells with a fixing agent in a low concentration and then, optionally, treatment with a reducing agent. A process for producing the same as well as a standard blood cell reagent for blood grouping and a method for blood grouping using the same are also disclosed. Further, the processed red blood cells sensitized with an antigen or antibody, a process for producing the same and a diagnostic method and a diagnostic reagent using the same are also disclosed.

## PROCESSED RED BLOOD CELLS AND PROCESS FOR PRODUCING THE SAME

The present invention relates to processed red blood cells and a process for producing the same. More particularly, it relates to processed red blood cells which have good antigen activity compared to that of natural red blood cells as well as long-term storage stability, and a process for producing the same.

Further, the present invention also relates to a method for blood grouping by using the processed red blood cells and a standard blood cell reagent for such a method. Furthermore, the present invention also relates to sensitized processed red blood cells obtained from the above processed red blood cells, a process for producing the same, a diagnostic method using the sensitized processed red blood cells, and a sensitized processed red blood cell reagent for such a method.

Usually, ABO blood grouping is carried out by using both "blood grouping test" wherein the blood group antigen of red blood cells to be tested is detected with standard sera, and so-called "back typing test" wherein anti A and anti B agglutinins in a serum to be tested are detected with known red blood cells of blood groups A and B.

However, there are many problems in such a "back typing test". For example, although live red blood cells are often used as the known red cells of blood groups A and B required for a "back typing test", their available period is very short such as for 3 to 4 weeks. Further, in a clinic or the like where fresh blood is hardly obtained, expensive standard blood cells should be purchased. These problems are obstructive to practice of a "back typing test" in every blood grouping.

Further, blood cells of blood group O wherein a lot of antigens such as Rh, Lewis, MNSs, P, Kell and the like are found are required for screening of antibodies which is regarded as an essential item to be tested before transfusion. However, in the blood cells used in this test, there is a same problem of storage stability as that in the above "back typing test", which is obstructive to practice of such a test in every antibody screening.

Furthermore, as a diagnostic method of infective diseases with bacteria, virus, rickettsia and the like, there is employed a method comprising sensitizing an antigen of these microorganisms to human or ovine red blood cells and mixing them with a serum to be tested to diagnose the infection based on agglutination. However, in this case, there is also such a problem that the available period of red blood cells is short and the cost thereof is expensive because live red blood cells are used.

As described above, the available period of natural red blood cells is short and their storage stability is insufficient and, therefore, natural red blood cells are not sufficiently utilized at present.

By the way, as processed red blood cells, there has been known fixed red blood cells which are obtained by treating natural red blood cells to provide storage stability.

That is, the processed red blood cells obtained by fixing red blood cells with formalin, glutaraldehyde and the like do not cause hemolysis and have long-term storage stability. And, they are also utilized as a carrier for indirect agglutination. See S. Avrameas, B. Taudou and S. Chuilon, Immunochemistry (6, 67 (1969)). For example, the processed red blood cells obtained by fixing human or ovine red blood cells have been used as a diagnostic agent for diagnosis of infective diseases and rheumatism by sensitizing antigen or antibody of bacteria or virus to the surfaces thereof and detecting the corresponding antibody or antigen based on agglutination of sensitized blood cells.

However, fixation which has been carried out to obtain such a carrier remarkably impairs original antigen activity (antigenicity) of red blood cells because considerably strong treatment, for example, treatment with aqueous 2.5% glutaraldehyde solution for one hour is effected. Thereby, such fixed red blood cells can not be used as standard red blood cells for detecting the antibody of corresponding blood group.

Accordingly, it is strongly requested to develop red blood cells which can be stored for a long period of time without impairment of their original antigenicity in medical field as well as other various fields.

Under these circumstances, the present inventors have studied intensively. As the result, it has been found that the above problems may be solved by fixing natural red blood cells under mild conditions and, optionally, treating them with a reducing agent to further recover agglutinability.

That is, the present invention desirably provides processed red blood cells having long-term storage stability while maintaining their original antigenicity.

Another desideratum of the present invention is to provide a process for producing the processed red blood cells.

It is also desirable that the present invention should provide a standard blood cell reagent for blood grouping containing the processed red blood cell.

Still further desiderata include:
  i) to provide a method for blood grouping using the processed red blood cells
  (ii) to provide an antigen or antibody sensitized processed red blood cells
  (iii) to provide a process for producing the antigen or antibody sensitized processed red blood cells
  (iv) to provide a diagnostic method using the sensitized processed red blood cells.
  (v) to provide a diagnostic reagent containing the sensitized processed red blood cells.

According to the present invention, there is provided processed red blood cells having storage stability while maintaining original antigenicity of natural red blood cells which are obtained by mild treatment of natural red blood cells with a fixing agent in a low concentration and then, optionally, treatment with a reducing agent.

The process for producing processed red blood cells of the present invention comprises subjecting natural red blood cells to mild treatment with a fixing agent in a low concentration and then, optionally, treatment with a reducing agent.

The standard blood cell reagent for blood grouping of the present invention comprises as an essential component processed red blood cells having storage stability with maintaining original antigenicity of natural red blood cells which are obtained by mild treatment of natural red blood cells with a fixing agent in a low concentration and then, optionally, treatment with a reducing agent.

The method for blood grouping of the present invention comprises detecting an antibody of a blood group by using processed red blood cells having storage stability with maintaining original antigenicity of natural red blood cells which are obtained by mild treatment of natural red blood cells with a fixing agent in a low concentration and then, optionally, treatment with a reducing agent.

The antigen or antibody sensitized processed red blood cells of the present invention are processed red blood cells having storage stability with maintaining original antigenicity of natural red blood cells which are obtained by mild treatment of natural red blood cells with a fixing agent in a low concentration and then, optionally, treatment with a reducing agent, and sensitization with an immunologically active material of the antigen or antibody.

The process for producing the antigen or antibody sensitized processed red blood cells of the present invention comprises sensitizing processed red blood cells having storage stability with maintaining original antigenicity of natural blood cells which are obtained by mild treatment of natural red blood cells with a fixing agent in a low concentration and then, optionally, treatment with a reducing agent, with an immunologically active material of the antigen or antibody.

The diagnostic method of the present invention comprises using the antigen or antibody sensitized processed red blood cells which are processed red blood cells having storage stability with maintaining original antigenicity of natural red blood cells obtained by mild treatment of natural red blood cells with a fixing agent in a low concentration and then, optionally, treatment with a reducing agent, and sensitization with an immunologically active material of the antigen or antibody.

The diagnostic reagent of the present invention comprises as an essential component the antigen or antibody sensitized processed red blood cells which are processed red blood cells having storage stability with maintaining original antigenicity of natural red blood cells obtained by mild treatment of natural red blood cells with a fixing agent in a low concentration and then, optionally, treatment with a reducing agent, and sensitization with an immunologically active material of the antigen or antibody.

Natural red blood cells used in the present invention are live red blood cells of human being, cattle, sheep, rabbit, fowl, neonatal chicken, goose, turkey, yellow-bellied terrapin and the like without treatment such as fixation or treatment with a reducing agent.

The concentration and kinds of a fixing agent which can be employed in the present invention include aldehydes such as 0.001 to 1% of glutaraldehyde (hereinafter abbreviated as GA), 0.01 to 5% of formaldehyde (hereinafter abbreviated as FA) and 0.01 to 5% of paraformaldehyde; imidates such as 0.001 to 1% of dimethylsuberoimidate (hereinafter abbreviated as DMS), 0.001 to 1% of dimethyladipoimidate (hereinafter abbreviated as DMA), 0.001 to 1% of dimethyl-3,3$'$-dithiobispropioimidate, 0.001 to 1% of N,N$'$-bis(2-carboxyimidoethyl)tartarimido dimethylester and 0.01 to 1% of 2-iminothiolane and 0.001 to 1% of methylbutyroimidate; isocyanates such as 0.01 to 1% of tolylene-2,4-diisocyanate, 0.01 to 1% of diisocyanic m-xylylene, 0.01 to 1% of phenylethylisocyanate, 0.01 to 1% of phenylethylisothiocyanate, 0.01 to 1% of hexamethylene diisocyanate and 0.01 to 1% of diphenylmethane diisocyanate; 0.0001 to 0.1% of tannic acid; 0.001 to 0.1% of osmic acid; 0.001 to 0.1% of chromic chloride; 0.001 to 1% of hydrogen peroxide; and the like.

Particularly, aldehydes such as 0.01 to 0.1% of GA, 0.01 to 2% of FA and 0.1 to 2% of paraformaldehyde; and imidates such as 0.01 to 0.1% of DMS, 0.01 to 0.1% of DMA and 0.01 to 0.1% of dimethyl-3,3'-dithiobispropioimidate are preferably used from the viewpoints that they are easily available, simply and safely handled and inexpensive. In the case of using processed red blood cells for detecting a blood group antibody, it is preferred to use them, particularly, at a lower concentration within the above range from the view point of maintaining antigenicity thereof.

The treatment with the fixing agent at a low concentration can be carried out easily. That is, in general, the treatment of red blood cells with the fixing agent is carried out at 4 to 40°C, preferably, 20 to 30°C for 1 to 10 minutes in a solution of the fixing agent within the above concentration range. This treatment is referred to as "mild treatment" in the present specification.

For example, in the case of using GA as the fixing agent, the treatment is carried out as follows:

Natural red blood cells are dispersed in 50 mM PBS (phosphate buffer saline) at pH 7.3 to prepare 25% dispersion. The same volume of 50 mM PBS containing 10 mM GA is then added to the resulting dispersion and the mixture is stirred thoroughly at 4 to 40°C, preferably 20 to 30°C for 1 to 3 minutes to effect the fixation reaction. Then, the fixation reaction is ceased by addition of 10 to 100 times volume of 50 mM PBS to the reaction solution and the resulting processed red blood cells are collected by centrifugation. They are washed thoroughly with 50 mM PBS to obtain the processed red blood cells of the present invention. Hereinafter, the processed red blood cells thus obtained only by mild fixation treatment are referred to as "semi-fixed processed red blood cells".

The mild fixation treatment of the present invention can give storage stability to red blood cells and, at the same time, impairment of antigenicity observed in a conventional strong fixation treatment, that is, impairment of agglutinability with an antibody can be prevented by the mild fixation treatment. Accordingly, agglutinability of the semi-fixed processed red blood cells of the present invention can be compared to that of natural red blood cells. However, rarely, agglutinability thereof is impaired by the mild treatment. In such a case, agglutinability can be recovered by treatment with a reducing agent.

Function and mechanism of the recovery of agglutinability by treatment with a reducing agent is not clear at present. However, as the reducing agent, for example, there can be used sodium sulfite, sodium boron hydride, lithium boron hydride, lithium aluminum hydride, hydrazine and the like.

The treatment with the reducing agent can be easily carried out and the conditions thereof can be suitably selected. In general, the treatment is carried out at 4 to 40°C, preferably, 20 to 37°C for 5 to 60 minutes. For example, in the case of using sodium sulfite, the treatment is carried out as follows:

Sodium sulfite is dissolved in 50 mM PBS to obtain 250 mM solution and then the above semi-fixed red blood cells are dispersed in the solution to obtain a 1 to 2% dispersion. The resulting dispersion is incubated at 4 to 40°C, preferably, 20 to 37°C for about 30 minutes and then processed red blood cells are collected by centrifugation. Then, they are washed thoroughly with 50 mM PBS to obtain processed red blood cells which is another aspect of the present invention. Hereinafter, the processed red blood cells thus obtained by subjecting to treatment with the reducing agent after the mild treatment are referred to as "recovered semi-fixed processed red blood cells".

Although the agglutinability recovery treatment with the reducing agent is preferably carried out subsequent to the mild treatment, but it may be carried out at 1 to 48 hours after the mild treatment.

By the way, it has been found that natural red blood cells treated with the reducing agent only or treated with the reducing agent and then the fixing agent also have storage stability with maintaining antigenicity.

Thus, usually, these processed red blood cells, that is, semi-fixed processed red blood cells and recovered semi-fixed processed red blood cells can be stored at 2 to 8°C in a storage solution. They can be stored stably for 1 to 3 months in a storage solution such as conventional Alsever's solution or the like.

At this time, in addition to the processed red blood cells, the present inventors have also succeeded in development of a storage solution for red blood cells having excellent storage stability. Such a novel storage solution comprises the ingredients of a conventional Alsever's solution and a water-soluble high molecular weight substance such as gelatin, gum arabic, starch, ficoll, polyvinyl pyrrolidone or dextran. Hitherto, in frozen storage of cells, red blood cells and the like, addition of gelatin has been known. However, gelatin has not been used with a preservative, antibiotic or the like in a diluted aqueous solution. The following Preparations illustrate the storage solution of the present invention.


Preparation 1

Preparation of gelatin type storage solution

The following ingredients were dissolved in water and the volume was adjusted to 1 liter with water to obtain the desired gelatin type storage solution.

| Ingredients | Amount (g) |
|---|---|
| Gelatin | 10 |
| Glucose | 18.66 |
| Sodium chloride | 4.18 |
| Sodium citrate | 8.0 |
| Adenine hydrochloride | 0.676 |
| Inosine | 2.68 |
| Kanamycin | 0.286 |

Preparation 2

Preparation of starch type storage solution

According to the same manner as described in Preparation 1, the desired starch type storage solution was prepared except that soluble starch was used instead of gelatin.

Preparation 3

Preparation of dextran type storage solution

According to the same manner as described in Preparation 1, the desired dextran type storage solution was prepared except that dextran was used instead of gelatin.

The above processed red blood cells can be stored stably for 6 months or more in the above storage solutions. For example, the following Table 1 shows changes of agglutination ability with time expressed by the maximum dilution which can cause agglutination in the case that semi-fixed processed red blood cells of the present invention obtained by fixing natural red blood cells with 5 mM GA for 3 minutes are stored at 4°C in Alsever's solution containing 1% gelatin, and in the case that the same natural red blood cells which are not subjected to fixation treatment are stored at 4°C in Alsever's solution.

## Table 1

| Experiment No. | Blood group | Natural red blood cells | | | Processed red blood cells | | |
|---|---|---|---|---|---|---|---|
| Storage period (months) | | 0 | 3 | 6 | 0 | 3 | 6 |
| 1 | A | 128 | 64 | 8 | 256 | 256 | 128 |
| 2 | B | 256 | 32 | haemolysis | 512 | 256 | 128 |
| 3 | O | 128 | 64 | 8 | 256 | 128 | 128 |
| 4 | B | 256 | 128 | 16 | 512 | 256 | 64 |
| 5 | B | 256 | 128 | haemolysis | 512 | 256 | 128 |
| 6 | A | 256 | 32 | 16 | 512 | 256 | 32 |
| 7 | A | 128 | 16 | 16 | 256 | 128 | 64 |
| 8 | B | 128 | 32 | haemolysis | 512 | 256 | 128 |

The above semi-fixed processed red blood cells and recovered semi-fixed processed red blood cells of the present invention is utilized in the standard blood cell reagent for blood grouping and the method for blood grouping of the present invention.

That is, like conventional live natural red blood cells, an antibody of a blood group can be detected by using the above semi-fixed processed red blood cells or recovered semi-fixed processed red blood cells by a "back typing test" of ABO blood group or an antibody screening according to a standard method.

For example, in a "back typing test", anti A and anti B agglutinins in serum can be detected readily according to a test tube method. That is, two drops of a serum to be tested are dropped in two test tubes, respectively. Then, one drop of A group processed red blood cells of the present invention is added dropwise to the serum in one test tube and one drop of B group processed red blood cells of the present invention is added dropwise to the serum in the other test tube. They are mixed and centrifuged. Then, the blood group can be readily judged by observing the presence of agglutination.

As a matter of course, in this blood grouping, there can be used the standard blood cell reagent for blood grouping of the present invention which is a stable form of the processed red blood cells of the present invention obtained by dispersing them in a conventional storage solution such as Alsever's solution, preferably the storage solution containing the above water-soluble high molecular weight substance to prepare a 1 to 3% dispersion.

Further, the above semi-fixed red blood cells and recovered semi-fixed red blood cells of the present invention is utilized in the processed red blood cells sensitized with an antigen or antibody of the present invention and a process for producing thereof as well as the diagnostic method and the diagnostic reagent using the sensitized processed red blood cells.

That is, the sensitized processed red blood cells of the present invention are obtained by sensitizing the above semi-fixed processed red blood cells or recovered semi-fixed recovery processed red blood cells with an immunologically active material according to a conventional method. By using the sensitized processed red blood cells as a carrier in indirect agglutination, infective diseases and autoimmune diseases can be diagnosed according to a conventional indirect agglutination. As the matter of course, in this diagnostic method, there can be used the sensitized processed red blood cells reagent of the present invention which is the storage stable form of the sensitized processed red blood cells of the present invention obtained by dispersing them in a storage solution, preferably, the storage solution containing the above water-soluble high molecular weight substance to prepare a 1 to 3% dispersion.

As the immunologically active material used for the above sensitization, for example, there are viral antigens such as those from hepatitis virus, rubella virus, measles virus, mumps virus, parainfluenza virus and the like; bacterial and fungal antigens such as those from Toxoplasma, Mycoplasma, Treponema pallidum and the like; antigens from hormones such as human chorionic gonadotropin (HCG), estrogen and the like; human immunoglobulin; rheumatoid factor; C-reactive protein and the like.

Examples of infective diseases wherein the diagnostic method of the present invention can be employed include tonsillitis, upper airway inflammation, pneumonia, pyoderma, erysipelas, acute glomerulonephritis, typhoid, paratyphoid, epidemic typhoid, pulsus fever, syphilis, mycoplasma diseases, toxoplasmosis, infectious mononucleosis, measles, rubella, influenza, viral hepatitis B and examples of autoimmune diseases include chronic rheumatism, systematic lupus erythematosus, chronic atrophic thyroiditis, Basedow's disease and the like.

For example, preparation of the sensitized processed red blood cells and the diagnosis method of the present invention can be carried out as follows:

Sensitization is carried out by adding tuberculin stock solution or purified polysaccharide antigen of tuberculin to 2% dispersion of the processed red blood cells of the present invention to obtain the sensitized processed red blood cells of the present invention.

The tuberculin sensitized processed red blood cells are added to a multiple dilution system of a patient's serum and they are thoroughly stirred. Agglutination titer can be measured by the maximum dilution which can cause agglutination. By assaying the antibody titer to tuberculin in a tuberculosis patient's, whether tuberculosis is active or not can be judged.

The processed red blood cells of the present invention, that is, the semi-fixed processed red blood cells and the recovered semi-fixed processed red blood cells have agglutinability compared to that of live red blood cells and excellent storage stability. Therefore, particularly, they are useful for detecting an antibody of a blood group. Then, the standard blood cell reagent of the processed red blood cells is also useful.

Further, the sensitized red blood cells of the present invention obtained by the processed red blood cells also have excellent storage stability and, when they are used as a carrier in indirect agglutination, they are useful for diagnosing infective diseases or autoimmune diseases because of very accurate test results.

The following Examples and Experiments further illustrate the present invention in detail but are not to be construed to limit the scope thereof.


Example 1

8

Human A group natural red blood cells were thoroughly washed with a physiological saline solution or PBS and then they were dispersed in PBS to prepare a 25% dispersion (5 ml), to which was added 50 mM PBS (pH 7.31, 5 ml) containing 10 mM GA. The mixture was thoroughly stirred at 25°C for one minute to effect a fixation reaction. Then, 50 mM PBS (pH 7.31, 500 ml) was added to the mixture to cease the reaction and the mixture was immediately centrifuged at 3,000 rpm for 10 minutes to recover a precipitate. The recovered precipitate was washed by centrifuging three times with 50 mM PBS (pH 7.31) to obtain processed A group red blood cells (semi-fixed processed red blood cells) of the present invention.

Examples 2 to 4

According to the same manner as described in Example 1, processed A group red blood cells were obtained except that 50 mM PBS (pH 7.31) containing the following fixing agent was used instead of 10 mM GA and the fixation time was 30 minutes instead of 1 minute.

| Example No. | Fixing agent |
|---|---|
| 2 | 0.6 M FA |
| 3 | 5 mM osmic acid |
| 4 | 2% paraformaldehyde |

Example 5

Human A group natural red blood cells were thoroughly washed with a physiological saline solution and then they were dispersed in the same solution to prepare a 50% dispersion (1 ml), to which was added 0.1 M sodium carbonate-physiological saline solution (10 ml) containing 2.5 mM DMS. The mixture was stirred at 37°C for 3 minutes to effect a fixation reaction. Then, 50 mM PBS (pH 7.31, 500 ml) containing 1% lysine is added to the mixture to cease the reaction and the mixture was immediately centrifuged at 3,000 rpm for 10 minutes to recover a precipitate. The recovered precipitate was washed by centrifuging with 50 mM PBS (pH 7.31) three times to obtain processed A group red blood cells of the present invention.

Example 6

According to the same manner as described in Example 5, processed B group red blood cells of the present invention were obtained except that human B group natural red blood cells was used as the raw material instead of human A group natural red blood cells and 1.5 mM DMA was used as the fixing agent instead of 2.5 mM DMS.

Examples 7 to 12

According to the same manner as described in Example 1, processed red blood cells of the present invention were obtained except that the following natural red blood cells and fixing agents were used and the following fixation time was employed.

| | | Natural RBC | Fixing agent | Fixation time |
|---|---|---|---|---|
| Ex. 7 | human B group RBC | | - | - |
| Ex. 8 | human B group RBC | | 0.6 M FA | 30 minutes |
| Ex. 9 | human O group RBC (D group (Rho(+) group)) | | - | - |
| Ex. 10 | human O group RBC (Rho(+) group) | | 5 mM osmic acid | 30 minutes |
| Ex. 11 | human O group RBC (N group) | | - | - |
| Ex. 12 | human O group RBC (M group) | | - | - |

(Note) RBC is Red blood cells. - represents the same conditions as those described in Example 1.

Example 13

According to the same manner as descried in Example 1, processed A group red blood cells (semi-fixed processed red blood cells) were obtained. The resulting processed red blood cells were dispersed in 50 mM PBS (pH 7.3) to prepare a 25% dispersion (1 ml), to which was added 50 mM PBS (pH 9, 10 ml) containing 250 mM sodium sulfite to effect an agglutinability recovery treatment at 37°C for 30 minutes. The red blood cells thus treated were washed thoroughly with 50 mM PBS to obtain processed A group red blood cells (recovered semi-fixed processed red blood cells) of the present invention.

Examples 14 to 16

According to the same manner as described in Examples 2, 6 and 9, respective semi-fixed processed red cells as described below were obtained. Then, according to the same manner as described in Example 13, a agglutinability recovery treatment was carried out with the reducing agent to obtain the processed red blood cells of the present invention.

| Example No. | Semi-fixed processed red blood cells |
|---|---|
| 14 | processed A group red blood cells of Ex. 2 |
| 15 | processed B group red blood cells of Ex. 6 |
| 16 | processed O group (Rho (+) group) red blood cells |

Examples 17 and 18

According to the same manner as described in Example 1, animal processed red blood cells (semi-fixed processed red blood cells) were obtained except that the following natural red blood cells were used as a raw material.

| Example No. | Natural red blood cells |
|---|---|
| 17 | ovine red blood cells |
| 18 | avian red blood cells |

Examples 19 and 20

According to the same manner as described in Example 13, animal processed red blood cells (recovered semi-fixed processed red blood cells) were obtained except that the following natural red blood cells were used as a raw material.

| Example No. | Natural red blood cells |
|-------------|-------------------------|
| 19 | ovine red blood cells |
| 20 | avian red blood cells |

Examples 21 to 32

The standard blood cell reagent for blood grouping of the present invention was prepared by dispersing the processed red blood cells obtained in Examples as shown in the following Table in the storage solution prepared in Preparations as shown in the following Table to obtain a 2% dispersion.

| Example No. | Processed red blood cells (Ex. No.) | Storage solution (Preparation No.) |
|-------------|-------------------------------------|-------------------------------------|
| 21 | 1 | 1 |
| 22 | 1 | 2 |
| 23 | 1 | 3 |
| 24 | 6 | 1 |
| 25 | 6 | 2 |
| 26 | 6 | 3 |
| 27 | 10 | 1 |
| 28 | 10 | 2 |
| 29 | 10 | 3 |
| 30 | 16 | 1 |
| 31 | 16 | 2 |
| 32 | 16 | 3 |

Examples 33 to 36

The standard blood cell reagent for blood grouping of the present invention was prepared by dispersing the processed red blood cells obtained in Examples as shown in the following Table in Alsever's solution, respectively to obtain a 2% dispersion.

| Example No. | Processed red blood cells (Example No.) |
|-------------|------------------------------------------|
| 33 | 1 |
| 34 | 6 |
| 35 | 10 |
| 36 | 16 |

Example 37

Blood grouping by "back typing test" was carried out by using the A group processed red blood cells (semi-fixed processed red blood cells) obtained in Example 1 and the B group processed red blood cells (semi-fixed processed red blood cells) obtained in Example 6 according to a test tube method.

That is, two drops of a serum (plasma) to be tested were dropped into two test tubes, respectively.

11

Then, one drop of A group processed red blood cells was added dropwise to the serum in one test tube and one drop of B group processed red blood cells was added dropwise to the serum in the other test tube. They were mixed and centrifuged at 3,400 rpm for 15 seconds. Then, the test tube was shaken gently and the presence of agglutination was observed and the result was recorded. The results for 30 samples of sera obtained by this operation are shown in Table 2. Further, the corresponding "blood grouping test" was carried out.

Table 2

| Sera No. | Agglutination | | Judgment by back typing test | Blood grouping test |
|---|---|---|---|---|
| | A group red blood cells | B group red blood cells | | |
| 1 | + | + | O | O |
| 2 | - | + | A | A |
| 3 | + | - | B | B |
| 4 | - | + | A | A |
| 5 | + | + | O | O |
| 6 | - | - | AB | AB |
| 7 | - | + | A | A |
| 8 | + | - | B | B |
| 9 | + | + | O | O |
| 10 | - | + | A | A |
| 11 | + | - | B | B |
| 12 | + | + | O | O |
| 13 | - | - | AB | AB |
| 14 | - | + | A | A |
| 15 | - | + | A | A |

Table 2 (continued)

| Sera No. | Agglutination | | Judgment by back typing test | Blood grouping test |
|---|---|---|---|---|
| | A group red blood cells | B group red blood cells | | |
| 16 | + | − | B | B |
| 17 | + | + | O | O |
| 18 | − | + | A | A |
| 19 | − | − | AB | AB |
| 20 | + | + | O | O |
| 21 | + | + | A | A |
| 22 | + | − | B | B |
| 23 | + | + | O | O |
| 24 | − | + | A | A |
| 25 | − | + | A | A |
| 26 | + | − | B | B |
| 27 | + | + | O | O |
| 28 | − | + | A | A |
| 29 | + | + | O | O |
| 30 | − | + | A | A |

(Note)    +:  agglutination

         −:  no agglutination

As seen from Table 2, the test results of the "back typing test" according to A and B group red blood cells were completely consistent with those of the "blood grouping test".

Further, blood grouping was carried out by using various combinations of A group semi-fixed processed red blood cells or A group recovered semi-fixed red blood cells obtained in other Examples, A group processed red blood cells reagent prepared from them and Alsever's solution or the storage solution containing the aforementioned water-soluble high molecular weight materials, and B group processed red blood cells or B group processed red blood cells reagent. In any case, the results were completely consistent with those of the blood grouping test.

Example 38

Detection of anti-D antibody by test tube centrifugation (I) (in the case of using semi-fixed processed red blood cells)

Detection of anti-D antibody in a serum of a subject was carried out by test tube centrifugation with human processed O and D (Rho (+)) group red blood cells obtained in Example 10 as follows:

One drop of processed red blood cells and one drop of 22% ovine serum albumin solution were added to each small test tube wherein one or two drops of the serum of the subject were placed and the contents were mixed with shaking the tube thoroughly. Then, the small test tube was centrifuged at 1,000 rpm at room temperature for 1 minutes and shaken gently to judge the presence of agglutination. For comparison, according to the same manner, agglutination was judged by using human natural O and D (Rho (+)) group red blood cells.

As seen from Table 3, in the case of using processed red blood cells of the present invention, the results of the judgment were the same as those obtained in the case of using natural red blood cells. The same results were obtained by using the reagent of the corresponding blood cells of Example 27.

Table 3

| Sera No. | Judgment according to natural O and D group red blood cells | Presence of anti-D antibody | Judgment according to processed red blood cells of the present invention | Presence of anti-D antibody |
|---|---|---|---|---|
| 1 | + | presence | + | presence |
| 2 | + | presence | + | presence |
| 3 | - | no | - | no |
| 4 | - | no | - | no |
| 5 | + | presence | + | presence |
| 6 | - | no | - | no |
| 7 | - | no | - | no |
| 8 | - | no | - | no |
| 9 | + | presence | + | presence |
| 10 | - | no | - | no |
| (Note) | | | | |

+ : agglutination

- : no agglutination

Example 39

Detection of anti-D antibody by test tube centrifugation (II) (in the case of using recovered semi-fixed processed red blood cells)

According to the same manner as described in Example 36, the same results of the judgment as that described in Table 3 were obtained by using processed O and D group red blood cells (recovered semi-fixed processed red blood cells) obtained in Example 16 and using 10 samples of the same sera of the subjects as those of Example 38. The same results were also obtained by using the corresponding blood cell reagent of Example 30.

Example 40

Preparation of processed red blood cells sensitized with rabbit γ-globulin (I)

Rabbit antiserum prepared by immunizing with ovine red blood cells was treated with heating at 56°C for 30 minutes to inactivate the complement activity. A part of the antiserum was diluted with a physiological saline solution to prepare 1/100 to 1/10,000 time diluted sera and each dilute serum (0.5 ml) and a 1% dispersion of ovine red blood cells (0.1 ml) were placed in a small test tube. Then, they were thoroughly mixed and allowed to stand at 37°C for 2 hours. The test tube was shaken gently and the presence of agglutination was observed. From the maximum dilution of the test tube wherein agglutination was observed, the agglutination titer of γ-globulin in rabbit antiserum was judged to be 1 : 4,000.

Further, to a 1/2,000 solution (20 ml) obtained by diluting the remaining rabbit antiserum wherein the complement activity was inactivated was added the dispersion (20 ml) of the processed ovine red blood cells (semi-fixed processed red blood cells) obtained in Example l7 in the gelatin type storage solution in Preparation 1, and they were thoroughly mixed in a beaker. Then, the mixture was allowed to stand at 25°C for one hour to obtain a 1% dispersion (40 ml) of processed red blood cells sensitized with rabbit γ-globulin of the present invention.

The dispersion can be used as the sensitized processed red blood cell reagent of the present invention.

Example 41

Preparation of processed red blood cells sensitized With rabbit γ-globulin (II)

According to the same manner as described in Example 17, the sensitized processed red blood cells of the present invention were obtained except that processed ovine red blood cells (recovered semi-fixed processed red blood cells) obtained in Example 19 were used instead of the processed ovine red blood cells (semi-fixed processed red blood cells) obtained in Example 17.

Experiment 1

Test I of rheumatoid factors (I)

10 Samples of sera of subjects were treated with heating at 56°C for 30 minutes to inactivate the complement activity. Each of them was diluted with the same amount of a physiological saline solution in the proportion of 1 : 2 to obtain a diluted serum (1 ml). To the diluted serum was added a precipitate of processed ovine red blood cells (0.1 ml) obtained in Example 17. The mixture was thoroughly mixed and allowed to stand overnight at 4°C. Then, it was centrifuged to obtain the 1 : 2 diluted serum wherein normal heterogeneous agglutinin was absorbed.

Then, the 1 : 2 diluted serum was diluted with physiological saline solution to prepare a multiple dilution system. Each diluted solution (0.5 ml) and sensitized processed red blood cell reagent (0.1 ml) composed of 1% processed red blood cells sensitized with rabbit γ-globulin obtained in Example 40 were placed in the small test tube. The mixture was thoroughly mixed and allowed to stand at 37°C for one hour and then at 4°C overnight. Then, it was incubated at 37°C for 15 hours, the test tube was shaken gently and the presence of agglutination was observed. The agglutination titer was corresponding to the maximum dilution wherein agglutination was observed.

For comparison, according to the same manner, the agglutination titer was obtained by using a dispersion of a 1% rabbit γ-globulin sensitized red blood cells prepared from natural ovine red blood cells as a raw material.

The results are shown in Table 4.

Table 4

| Sera No. | Agglutination titer in case of using a dispersion of sensitised natural ovine red blood cells | Agglutination titer in case of using sensitized processed ovine red blood cells reagent |
|---|---|---|
| 1 | 1 : 64 | 1 : 64 |
| 2 | 1 : 2 | 1 : 2 |
| 3 | 1 : 16 | 1 : 32 |
| 4 | 1 : 4 | 1 : 4 |
| 5 | 1 : 64 | 1 : 64 |
| 6 | 1 : 32 | 1 : 64 |
| 7 | 1 : 16 | 1 : 32 |
| 8 | 1 : 4 | 1 : 4 |
| 9 | 1 : 2 | 1 : 2 |
| 10 | 1 : 16 | 1 : 16 |

As seen from Table 4, in the case of using sensitized processed red blood cell reagent of the present invention, the same test results as those obtained in the case of using the dispersion of sensitized natural red blood cells having the same concentration were obtained.

Experiment 2

Test of rheumatoid factors (II)

According to the same manner as described in Experiment 1, a test was carried out except that the reagent of the red blood cells sensitized with rabbit γ-globulin (recovered sensitized semi-fixed processed red blood cells) obtained in Example 41 was used instead of the reagent of the red blood cells sensitized with rabbit γ-globulin (sensitized semi-fixed processed red blood cells) obtained in Example 40. Like Experiment 1, the same results as those obtained in the case of using the dispersion of sensitized natural red blood cells having the same concentration were obtained.

Example 42

Preparation of processed red blood cells sensitized with tuberculin polysaccharide (I)

Human O group processed red blood cells (semi-fixed processed red blood cells) obtained in Example 9 were dispersed in 50 mM PBS (pH 7.3) to obtain a 50% dispersion. To 1 ml of the dispersion was added 1 ml of tuberculin polysaccharide (manufactured by Pasteur Laboratories). Then, it was incubated at 37°C for 2 hours to sensitize tuberculin polysaccharide to the red blood cells and washed thoroughly with 50 mM PBS (pH 7.3). Then, it was dispersed in the gelatin type storage solution prepared in Preparation 1 to obtain a 0.25% dispersion of the processed red blood cells sensitized with tuberculin polysaccharide of the present invention.
This can be used as the sensitive processed red blood cell reagent of the present invention.

Example 43

Preparation of processed red blood cells sensitized with tuberculin polysaccharide (II)

According to the same manner as described in Example 42, a 0.25% dispersion of the processed red blood cells sensitized with tuberculin polysaccharide of the present invention was obtained except that human O group processed red blood cells (recovered semi-fixed processed red blood cells) obtained in Example 16 were used instead of human O group processed red blood cells (semi-fixed processed red blood cells) obtained in Example 9.

Experiment 3

Detection of anti-tubercle bacilli antibody (I)

Anti-tubercle bacilli antibody in a serum of a subject was detected by using the reagent of the processed red blood cells sensitized with tuberculin polysaccharide obtained in Example 42 as follows:

10 Samples of sera were inactivated at 50°C for 30 minutes and diluted with a physiological saline solution to prepare a multiple dilution system. The reagent of 0.25% processed red blood cells sensitized with tuberculin polysaccharide (0.1 ml) obtained in Example 42 was added to each diluted serum (0.5 ml) as described above in the small test tube, and the tube was thoroughly mixed and allowed to stand overnight at 25 to 30°C. Then, the small test tube was shaken gently and the presence of agglutination was observed. The agglutination titer was corresponding to the maximum dilution wherein agglutination was observed.

For comparison, according to the same manner as described above, the agglutination titer was determined by using the dispersion of 0.25% natural red blood cells sensitized with tuberculin polysaccharide obtained by sensitizing human O group red blood cells.

The results are shown in Table 5

Table 5

| Sera No. | Agglutination titer in case of using a dispersion of sensitised natural humane O group red blood cells | Agglutination titer in case of using sensitized processed humane O group red blood cells |
|---|---|---|
| 1 | 1 : 16 | 1 : 16 |
| 2 | negative | negative |
| 3 | 1 : 32 | 1 : 32 |
| 4 | 1 : 16 4 | 1 : 8 |
| 5 | 1 : 32 | 1 : 32 |
| 6 | negative | negative |
| 7 | 1 : 32 | 1 : 32 |
| 8 | 1 : 16 | 1 : 8 |
| 9 | negative | negative |
| 10 | 1 : 16 | 1 : 16 |

As seen from Table 5, in the case of using the sensitized processed red blood cell reagent of the present invention, the same test results as those obtained in the case of using the dispersion of sensitized natural red blood cells having the same concentration were obtained.

Experiment 4

Detection of anti-tubercle bacilli antibody (II)

According to the same manner as described in Experiment 3, a test was carried out except that the reagent of the processed red blood cells sensitized with tuberculin polysaccharide (sensitized recovered semi-fixed processed red blood cells) obtained in Example 43 were used instead of the reagent of the processed red blood cells sensitized with tuberculin polysaccharide (sensitized semi-fixed processed red blood cells) obtained in Example 42. Like Experiment 3, the same results as those obtained in the case of using the dispersion of sensitized natural red blood cells having the same concentration were obtained.

Experiment 5

17

Detection of cold agglutinin

Cold agglutinin in a serum of a subject was detected by using the human O group red blood cells prepared in Example 10. That is, 10 samples of sera were diluted with physiological saline solution to prepare a multiple dilution system. To a small test tube wherein 0.5 ml of a diluted serum was placed was added 0.25% human O group processed red blood cells (0.5 ml) and they were thoroughly mixed. Then, they were allowed to stand overnight at 0 to 5°C and the presence of agglutination was observed at 20°C or lower. The agglutination titer was corresponding to the maximum dilution of the small test tube wherein agglutination was observed.

After judgment of agglutination, when the small test tube was incubated at 37°C for 30 minutes, agglutination was disappeared. Thus, it has been found that agglutination is caused due to cold agglutinin.

The results are shown in Table 6

Table 6

| Sera No. | Agglutination titer in case of using natural red blood cells | Agglutination titer in case of processed red blood cells |
|---|---|---|
| 1 | 1 : 4 | 1 : 4 |
| 2 | 1 : 8 | 1 : 8 |
| 3 | negative | negative |
| 4 | 1 : 2 | 1 : 2 |
| 5 | 1 : 16 | 1 : 16 |
| 6 | 1 : 4 | 1 : 4 |
| 7 | negative | negative |
| 8 | 1 : 8 | 1 : 8 |
| 9 | 1 : 2 | 1 : 2 |
| 10 | 1 : 4 | 1 : 4 |

As seen from Table 6, the agglutination titer of cold agglutinin in the serum of the subject gave the same results as those obtained in the case of using natural human O group red blood cells.

**Claims**

1. A process for producing processed red blood cells having storage stability and maintained original antigenicity of natural red blood cells which comprises subjecting natural red blood cells to mild treatment with a fixing agent in a low concentration.

2. A process according to claim 1, wherein the fixing agent in a low concentration is selected from the group consisting of 0.001 to 1% of glutaraldehydes, 0.01 to 5% of formaldehyde and 0.01 to 5% of paraformaldehyde, 0.001 to 1 % of dimethylsuberoimidate, 0.001 to 1% of dimethyladipoimidate, 0.001 to 1% of dimethyl-3,3'- dithiobispropiomidate, 0.001 to 1% of N,N'-bis(2-carboxyimidoethyl)tartarimido dimethylester, 0.001 to 1% of 2-iminothiolane, 0.001 to 1% of methylbutyroimidate, 0.01 to 1% of tolylene-2,4-diisocyanate, 0.01 to 1% of diisocyanic m-xylylene, 0.01 to 1% of phenylethylisocyanate, 0.01 to 1% of phenylethylisothiocyanate 0.01 to 1% of hexamethylene diisocyanate, 0.01 to 1% of diphenylmethane diisocyanate, 0.0001 to 0.1% of tannic acid, 0.001 to 0.1% of osmic acid, 0.001 to 0.1% of chrome chloride and 0.0001 to 1% hydrogen peroxide.

3. A process according to claim 1 or claim 2 further comprising treatment of the natural red blood cells with a reducing agent.

4. A process according to claim 3 wherein the reducing agent is selected from sodium sulfite, sodium borohydride, lithium borohydride, lithium aluminium hydride and hydrazine.

5. Processed red blood cells having storage stability and maintained original antigenicity of natural red blood cells as obtained by the method of any one of claims 1 to 4.

6. A standard blood cell reagent for blood grouping which comprises processed red blood cells according to claim 5.

7. A method for blood grouping which comprises detecting an antibody of a blood group by using processed red blood cells according to claim 5.

8. Antigen or antibody sensitized processed red blood cells which are processed red blood cells according to claim 5 sensitized with an immunologically active material of the antigen or antibody.

9. A process for producing antigen or antibody which comprises sensitizing processed red blood cells according to claim 5 with an immunologically active material of the antigen or antibody.

10. A process according to claim 9, wherein the immunologically active material is selected from antigens and antibodies of virus, toxoplasma, mycoplasma, bacteria and fungi, hormones, human immunoglobulins, nucleic acids, histone, thryoglobulin and C-reactive protein.

11. A diagnostic method which comprises using antigen or antibody sensitized processed red blood cells according to claim 8.

12. A method according to claim 11, wherein the method is used for diagnosis of infective diseases selected from tonsillitis, upper airway inflammation pneumonia, pyoderma, erysipelas, acute glomerulonephritis, typhoid, paratyphoid, epidemic typhoid, pulsus fever, syphilis, mycoplasma diseases, toxoplasmosis, infectious mononucleosis, measles, rubella, influenza and viral hepatitis type B.

13. A method according to claim 11, wherein the method is used for diagnosis of autoimmune diseases selected from chronic rheumatism, systematic lupus erythematosus, chronic atrophic thryoiditis and Basedow's disease.

14. A diagnostic sensitized processed red blood cell reagent which comprises antigen or antibody sensitized processed red blood cells according to claim 8.